# DEMANDE DE BREVET EUROPEEN

(11) **EP 1 566 171 A1**
(43) Date de publication de la demande: **24.08.2005**
(21) Numéro de dépôt: 05290081.8
(22) Date de dépôt: 13.01.2005
(51) Int. Cl.: A61K 7/48

(54) **Utilisation d'un tétrapolymère acrylique comme agent tenseur de la peau.**

(30) Priorité: 23.02.2004 FR 0450333
(71) Demandeur: L'OREAL, 75008 Paris (FR)
(72) Inventeur: Charbit, Yael, 94700 Maisons-Alfort (FR)
(74) Mandataire: Renard, Emmanuelle

(57) **Abrégé**

La présente invention se rapporte à un procédé cosmétique de traitement d'une peau humaine ridée, comprenant l'application topique sur ladite peau d'une composition renfermant, dans un milieu physiologiquement acceptable, un tétrapolymère d'acide méthacrylique, de méthacrylate de méthyle, d'acrylate de butyle et de (méth)acrylate d'alkyles en C₁₆-C₂₀, ladite composition étant exempte de filtres solaires.

Elle se rapporte également à l'utilisation cosmétique d'un tétrapolymère d'acide méthacrylique, de méthacrylate de méthyle, d'acrylate de butyle et de (méth)acrylate d'alkyles en C₁₆-C₂₀ en tant qu'agent tenseur de la peau humaine.

## Description

La présente invention se rapporte à un procédé cosmétique de traitement d'une peau humaine ridée, comprenant l'application topique sur ladite peau d'une composition renfermant, dans un milieu physiologiquement acceptable, un tétrapolymère d'acide méthacrylique, de méthacrylate de méthyle, d'acrylate de butyle et de (méth)acrylate d'alkyles en C₁₆-C₂₀, ladite composition étant exempte de filtres solaires.

Elle se rapporte également à l'utilisation cosmétique d'un tétrapolymère d'acide méthacrylique, de méthacrylate de méthyle, d'acrylate de butyle et de (méth)acrylate d'alkyles en C₁₆-C₂₀ en tant qu'agent tenseur de la peau humaine.

Les femmes, voire même les hommes, ont tendance actuellement à vouloir paraître jeunes le plus longtemps possible et cherchent par conséquent à estomper les marques du vieillissement de la peau, qui se traduisent notamment par des rides et des ridules. A ce sujet, la publicité et la mode font état de produits destinés à garder le plus longtemps possible une peau éclatante et sans ride, marques d'une peau jeune, d'autant plus que l'aspect physique agit sur le psychisme et/ou sur le moral.

Jusqu'à présent, on traitait les rides et les ridules à l'aide de produits cosmétiques contenant des actifs agissant sur la peau, par exemple en l'hydratant ou en améliorant son renouvellement cellulaire ou encore en favorisant la synthèse, ou en prévenant la dégradation, des fibres élastiques qui composent le tissu cutané.

Ces produits ont toutefois l'inconvénient de n'être efficaces pour le traitement des rides qu'après un certain temps d'application. Or, on cherche de plus en plus à disposer de produits anti-rides à un effet immédiat.

A cette fin, il a été proposé depuis quelques années des agents à effet tenseur qui lissent immédiatement après application les rides et ridules et contribuent à atténuer les marques de fatigue. Ces composés agissent en formant un film qui, au séchage, provoque la rétraction du stratum corneum, la couche cornée superficielle de l'épiderme.

Parmi ces agents tenseurs, il a été proposé d'utiliser des protéines végétales. Toutefois, celles-ci posent des problèmes de formulation (perte de viscosité, problèmes de stabilité) et ne sont pas toujours très cosmétiques, en ce sens qu'elles confèrent un toucher collant aux compositions les contenant, voire produisent une odeur et/ou une couleur désagréables.

Il a par ailleurs été proposé dans la demande EP-0 944 381 d'utiliser comme agents tenseurs des latex constitués d'homo- et copolymères acryliques. Toutefois, parmi ces polymères, tous ne permettent pas la réalisation de compositions cosmétiques stables, en particulier lorsque ces compositions sont formulées sous forme d'émulsions. Des règles de formulation contraignantes doivent dans ce cas être respectées. En outre, la Demanderesse a découvert que la présence d'un tensioactif, généralement nécessaire à la stabilisation des émulsions, avait un impact négatif sur le pouvoir tenseur exercé par ces polymères.

Il reste donc le besoin de disposer de composés ayant des effets tenseurs au moins aussi importants que ceux de l'art antérieur, qui soient faciles à formuler sous forme de compositions stables et qui présentent de bonnes propriétés cosmétiques rendant ces compositions agréables à utiliser.

Or, la Demanderesse a découvert, de manière surprenante et inattendue, que certains tétrapolymères acryliques permettaient de satisfaire ce besoin. En particulier, ces polymères permettent non seulement de tendre efficacement la peau, et de masquer ainsi les rides et ridules et les signes de fatigue, mais ils ont également des propriétés auto-émulsionnantes permettant de les formuler en émulsion sans avoir recours à un tensioactif, et d'obtenir ainsi facilement des compositions stables et ayant un bon effet tenseur.

Ces polymères ont déjà été décrits comme étant utiles pour améliorer la résistance à l'eau et/ou à l'abrasion de compositions (US 2003/0021847) telles que des compositions solaires (WO 03/082237) et pour éviter le peluchage de celles-ci (demande américaine n° 10/372330). Il a également été suggéré qu'ils pouvaient prévenir le dessèchement cutané et possédaient des propriétés anti-inflammatoires en raison de leur capacité à structurer les lipides épidermiques et le sébum et à renforcer ainsi la barrière lipidique dans le stratum corneum (WO 03/103615).

Toutefois, à la connaissance de la Demanderesse, il n'a encore jamais été suggéré que ces polymères avaient la capacité de tendre la peau et que l'on pouvait ainsi envisager de les utiliser pour lisser les rides.

La présente invention a donc pour objet un procédé cosmétique de traitement d'une peau humaine ridée, comprenant l'application topique sur ladite peau d'une composition renfermant, dans un milieu physiologiquement acceptable, un tétrapolymère d'acide méthacrylique, de méthacrylate de méthyle, d'acrylate de butyle et de (méth)acrylate d'alkyles en C₁₆-C₂₀, ladite composition étant exempte de filtres solaires.

Elle a aussi pour objet l'utilisation cosmétique d'un tétrapolymère d'acide méthacrylique, de méthacrylate de méthyle, d'acrylate de butyle et de (méth)acrylate d'alkyles en C₁₆-C₂₀ en tant qu'agent tenseur de la peau humaine, en particulier en vue de lisser les rides et/ou estomper les signes cutanés de fatigue.

Le tétrapolymère utilisé selon l'invention comprend de préférence : de 0,5 à 10% en poids, avantageusement de 1 à 2% en poids, d'acide méthacrylique ; de 25 à 90% en poids, avantageusement de 10 à 40% en poids, de méthacrylate de méthyle ; de 10 à 90% en poids, avantageusement de 30 à 50% en poids, d'acrylate de butyle ; et de 25 à 75% en poids, avantageusement de 25 à 35% en poids, de (méth)acrylate d'alkyles en C₁₆-C_{20.} Par "(méth)acrylates d'alkyles en C₁₆-C₂₀, on entend un ou plusieurs monomères constitués de (méth)acrylate d'alkyle dont la chaîne alkyle renferme de 16 à 20 atomes de carbone, de préférence un mélange de monomères constitué de (meth)acrylate d'alkyles en C₁₆, C₁₈ et C₂₀. Les (méth)acrylates d'alkyles comprennent de préférence de 25 à 35% en poids de méthacrylate de cétyle, de 55 à 65% en poids d'acrylate de stéaryle et de 5 à 15% en poids d'acrylate d'eicosyle.

Des tétrapolymères tels que définis précédemment, ainsi que leur procédé de préparation, sont en particulier décrits dans la demande US 2003/0021847.

Ils peuvent être préparés par polymérisation en émulsion des monomères ci-dessus en présence d'un initiateur de radicaux libres tel que le peroxyde d'hydrogène, le tert-butyl hydroperoxyde, ou le persulfate de sodium, potassium, lithium ou ammonium, l'initiateur étant éventuellement combiné à un agent réducteur pour former un système redox et à un catalyseur constitué d'un métal de transition tel qu'un sel de cuivre ou de fer. La réaction peut par exemple être effectuée à une température comprise entre 10 et 120°C, de préférence voisine de 85°C, pendant une durée approximative de trois heures.

Un tétrapolymère de ce type est en particulier disponible auprès de la société ROHM & HAAS sous la dénomination commerciale Allianz OPT sous forme de dispersion hydro-glycolique à 48% de matière active.

La quantité de tétrapolymère incorporée dans la composition selon l'invention peut varier dans une large mesure en fonction de l'effet recherché. Pour donner un ordre de grandeur, la composition peut renfermer de 0,1 à 10% en poids, et de préférence de 0,2 à 5% en poids, de tétrapolymère, par rapport au poids total de la composition.

Cette composition est généralement adaptée à une application topique sur la peau et comprend donc généralement un milieu physiologiquement acceptable, c'est-à-dire compatible avec la peau et/ou ses phanères. Il s'agit de préférence d'un milieu cosmétiquement acceptable, c'est-à-dire qui présente une couleur, une odeur et un toucher agréables et qui ne génère pas d'inconforts inacceptables (picotements, tiraillements, rougeurs), susceptibles de détourner la consommatrice d'utiliser cette composition.

Cela est particulièrement important lorsque la composition utilisée selon l'invention est destinée à être appliquée autour des yeux, ce qui constitue un mode de réalisation avantageux de la présente invention.

La composition selon l'invention peut en outre se présenter sous toutes les formes galéniques classiquement utilisées pour une application topique et notamment sous forme de dispersions du type lotion ou gel aqueux, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de dispersions vésiculaires de type ionique et/ou non ionique, ou de dispersions cire/phase aqueuse. Ces compositions sont préparées selon les méthodes usuelles.

Selon un mode préféré de réalisation de l'invention, la composition se présente sous forme d'une émulsion H/E.
Comme huiles utilisables dans la composition selon l'invention, on peut citer :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone ou encore, par exemple, les huiles végétales telles que l'huile d'amande d'abricot et l'huile de beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone ;
- les hydrocarbures linéaires ou ramifiés, d'origine minérale ou synthétique, tels que les huiles de paraffine, volatiles ou non, et leurs dérivés, l'isohexadecane, l'isododecane, la vaseline, les polydécènes, le polyisobutène hydrogéné tel que l'huile de Parléam® ;
- des huiles essentielles naturelles ou synthétiques ;
- les alcools gras ayant de 8 à 26 atomes de carbone, comme l'alcool cétylique, l'alcool stéarylique et leur mélange (alcool cétylstéarylique) ou l'octyldodécanol ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que le cyclohexasiloxane et le cyclopentasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphénylsiloxanes, les diphényl-diméthicones, les diphénylméthyldiphényl trisiloxanes, les 2-phényléthyltriméthyl-siloxysilicates, et les polyméthylphénylsiloxanes ; et
- leurs mélanges.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila ; et les gommes telles que les gommes de silicone (diméthiconol).

Ces corps gras peuvent être choisis de manière variée par l'homme du métier afin de préparer une composition ayant les propriétés, par exemple de consistance ou de texture, souhaitées.

Cette composition peut en outre contenir divers adjuvants couramment utilisés dans le domaine cosmétique, tels que des charges ; des conservateurs ; des séquestrants ; des colorants ; des parfums.

Comme charges, on peut citer par exemple, les particules de polyamide (Nylon) sous forme sphérique ou sous forme de microfibres ; les microsphères de polyméthacrylate de méthyle ; les poudres de copolymère éthylène-acrylate ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinique ; les microbilles de résine de silicone telles que celles commercialisées sous la dénomination TOSPEARL par la société Toshiba Silicone ; la silice ; les oxydes métalliques tels que le dioxyde de titane ou l'oxyde de zinc ; le mica ; et leurs mélanges.

Bien entendu, l'homme du métier veillera à choisir ce ou ces éventuels composés additionnels et/ou leur quantité de manière telle que les propriétés avantageuses de la composition selon l'invention ne soient pas, ou substantiellement pas, altérées par l'adjonction envisagée.

En particulier, selon un mode de réalisation avantageux, la composition utilisée selon l'invention ne comprend sensiblement pas de tensioactif, c'est-à-dire moins de 1% en poids, voire moins de 0,5% en poids, de tensioactif, par rapport au poids total de la composition. Par "tensioactif", on entend tout composé ou mélange de composés identifiés comme tels dans l'un au moins des deux ouvrages suivants : Mc Cutcheon's : Emulsifiers and Détergents, International Edition, et International Cosmetic Ingredient Dictionary and Handbook (CTFA).

Il a en effet été mis en évidence par la Demanderesse que le tétrapolymère selon l'invention permettait la réalisation de compositions stables sans nécessiter le recours à un tensioactif ni même à des épaississants.

Toutefois, pour améliorer la stabilité de telles compositions, en particulier lorsque le tétrapolymère n'est présent qu'à hauteur de 4% en poids ou moins (en matière active) dans la composition, il peut être utile de l'associer à au moins un épaississant, qui peut par exemple être choisi parmi : les homo- et copolymères d'acide acrylique (tels que les carbomers et les copolymères commercialisés sous les dénominations Pemulen TR1 et Pemulen TR2 par NOVEON), et les homo- et copolymères d'acrylamide et/ou d'acide 2-acrylamido-2-méthylpropane sulfonique (AMPS), tels que ceux commercialisés sous les dénominations Sepigel 305 et Simulgel 600 par SEPPIC et sous les dénominations Hostacerin AMPS et Aristoflex HMS par CLARIANT.

En outre, pour renforcer les effets anti-âge de la composition utilisée selon l'invention, il pourra être avantageux d'introduire dans cette composition au moins un composé choisi parmi : les agents desquamants ; les agents hydratants ; les agents dépigmentants ou propigmentants ; les agents anti-glycation ; les inhibiteurs de NO-synthase ; les agents stimulant la synthèse de macromolécules dermiques ou épidermiques et/ou empêchant leur dégradation ; les agents stimulant la prolifération des fibroblastes et/ou des kératinocytes ou stimulant la différenciation des kératinocytes ; les agents dermo-décontractants et/ou myorelaxants ; les agents tenseurs ; les agents anti-pollution et/ou anti-radicalaires ; les agents agissant sur la microcirculation ; les agents agissant sur le métabolisme énergétique des cellules ; et leurs mélanges.

Des exemples de tels composés additionnels sont : le rétinol et ses dérivés tels que le palmitate de rétinyle ; l'acide ascorbique et ses dérivés tels que l'ascorbyl phosphate de magnésium et le glucoside d'ascorbyle ; le tocophérol et ses dérivés tels que l'acétate de tocophéryle ; l'acide nicotinique et ses précurseurs tels que la nicotinamide ; l'ubiquinone ; le glutathion et ses précurseurs tels que l'acide L-2-oxothiazolidine-4-carboxylique ; les extraits de plantes et notamment les protéines végétales et leurs hydrolysats, ainsi que les phytohormones ; les extraits marins tels que les extraits d'algues ; les extraits bactériens ; les sapogénines telles que la diosgénine et les extraits de Wild Yam en contenant ; les céramides ; les α-hydroxyacides ; les β-hydroxyacides, tels que l'acide salicylique et l'acide n-octanoyl-5-salicylique ; le resvératrol ; les oligopeptides et pseudodipeptides et leurs dérivés acylés ; les sels de manganèse et de magnésium , en particulier les gluconates ; et leurs mélanges.

L'invention sera maintenant illustrée par les exemples non limitatifs suivants. Dans ces exemples, les quantités sont indiquées en pourcentage pondéral, sauf indication contraire.

### EXEMPLES

### Exemple 1 : Démonstration de l'effet tenseur in vivo

On a préparé une émulsion H/E ayant la composition ci-dessous, de façon classique pour l'homme du métier.

| | |
|---|---|
| Glycérine | 5 % |
| Conservateurs | 0,4 % |
| Copolymère acrylique ⁽¹⁾ | 1 % |
| Neutralisant | 0,05 % |
| Dispersion aqueuse de tétrapolymère | |
| acrylique ⁽²⁾ | 2 % |
| Copolymère d'AMPS ⁽³⁾ | 0,3 % |
| Polyisobutène hydrogéné | 3 % |
| Cyclohexasiloxane | 4 % |
| Isohexadécane | 3 % |
| Parfum qs | |
| Eau qsp | 100 % |

| | |
|---|---|
| (1) Synthalen W2000 de SIGMA (copolymère acrylates / palmeth-25 acrylate) | |
| (2) Allianz OPT de ROHM & HAAS (48% de matière active) | |
| (3) Aristoflex HMS de CLARIANT (copolymère AMPS / steareth-25 methacrylate réticulé) | |

Cette composition a été appliquée sur un panel de cinq femmes présentant des rides et ridules. Elle a été appliquée précisément sur la moitié du visage (et le contour de l'oeil correspondant) et l'état de la peau a été évalué immédiatement après application, par comparaison avec l'autre moitié du visage (non traitée).

Trois femmes sur cinq ont jugé que cette composition produisait un effet lissant moyen ou léger sur le visage, qui semblait plus lisse et reposé, et un effet tenseur moyen ou léger autour de l'oeil, qui conduisait à un estompage des ridules.

Il a par ailleurs été vérifié in vitro que le copolymère d'AMPS et le copolymère acrylique ne présentaient pas d'effet tenseur perceptible aux taux indiqués dans la formule ci-dessus.

Cet exemple démontre donc la pouvoir tenseur du tétrapolymère utilisé selon l'invention.

### Exemple 2 : Démonstration de l'effet tenseur in vivo

L'effet tenseur de deux polymères acryliques a été évalué in vitro, en utilisant une solution à 0,96% de matière active, et rapporté à celui du tétrapolymère selon l'invention, auquel la valeur 100 a été attribuée. Les résultats sont présentés dans le Tableau 1 ci-dessous.

**Tableau 1**

| Polymère acrylique | Effet tenseur |
|---|---|
| Tétrapolymère acrylique selon l'invention (Allianz OPT de ROHM & HAAS) | 100 % |
| Copolymère acide acrylique / acrylate d'éthyle (Aculyn 33 de ROHM & HAAS) | 94 % |
| Polymère acrylique (Carbopol Aqua SF1 de NOVEON) | 83 % |

Ces résultats montrent que le tétrapolymère selon l'invention présente de meilleures propriétés de tension de la peau que d'autres polymères acryliques

### Exemple 3 : Démonstration de la stabilité des émulsions

On a préparé trois émulsions H/E désignées ci-après par émulsions A, B et C, ayant la composition ci-dessous, dans lesquelles trois polymères acryliques différents ont été respectivement incorporés.

| | |
|---|---|
| Alcool | 10 % |
| Polyisobutène hydrogéné | 10 % |
| Glycérine | 5 % |
| Cyclohexasiloxane | 5 % |
| Polymère acrylique | 4,6 % (matière active) |
| Conservateurs | 0,4 % |
| Parfum qs | |
| Eau qsp | 100 % |

Ces compositions ont été préparées de la manière suivante : le polyisobutène hydrogéné et le cyclohexasiloxane ont été dispersés à température ambiante, au mixer, dans le mélange d'eau, de polymère acrylique, de glycérine et de conservateurs, puis l'alcool et le parfum ont été ajoutés à la même température.

La stabilité de ces émulsions a été évaluée à l'oeil nu. Les résultats sont rassemblés dans le Tableau 2 ci-après.

**Tableau 2**

| Emulsion | Polymère acrylique | Stabilité | Résultat cosmétique |
|---|---|---|---|
| A | Tétrapolymère acrylique selon l'invention (Allianz OPT de ROHM & HAAS) | Formule d'aspect homogène, lait très fluide. Pas de déphasage. | Ne peluche pas à l'application. Frais, glissant. Riche et filmogène au final. |
| B | Copolymère acide acrylique / acrylate d'éthyle (Aculyn 33 de ROHM & HAAS) | Relargage. Amas de phase grasse gélifiée en surface. | Impossible à évaluer (émulsion déphasée) |
| C | Polymère acrylique (Carbopol Aqua SF1 de NOVEON) | Formule déphasée, mélange hétérogène comportant une phase grasse gélifiée en surface. Relargage important. | Impossible à évaluer (émulsion déphasée) |

Ces résultats montrent que le tétrapolymère selon l'invention permet la formulation d'émulsions stables sans tensioactif. Dans la mesure où les tensioactifs ne sont pas toujours bien tolérés par les peaux les plus sensibles, ce tétrapolymère est donc bien adapté à une utilisation sur des peaux sensibles présentant des signes de vieillissement et/ou de fatigue.

En outre, la possibilité de le formuler sans tensioactif permet au tétrapolymère selon l'invention d'être formulé avec un minimum de contraintes dans des supports cosmétiques très variés, puisqu'il ne sera pas nécessaire de tenir compte des incompatibilités chimiques entre certains tensioactifs et d'autres constituants de ces supports. Ce tétrapolymère offre donc une grande latitude de formulation.

## Revendications

1. Procédé cosmétique de traitement d'une peau humaine ridée, comprenant l'application topique sur ladite peau d'une composition renfermant, dans un milieu physiologiquement acceptable, un tétrapolymère d'acide méthacrylique, de méthacrylate de méthyle, d'acrylate de butyle et de (méth)acrylate d'alkyles en C₁₆-C₂₀, ladite composition étant exempte de filtres solaires.

2. Procédé selon la revendication 1, **caractérisé en ce que** ledit tétrapolymère comprend : de 1 à 2% en poids d'acide méthacrylique, de 10 à 40% en poids de méthacrylate de méthyle, de 30 à 50% en poids d'acrylate de butyle, et de 25 à 35% en poids de (méth)acrylate d'alkyles en C₁₆-C₂₀.

3. Procédé selon la revendication 1 ou 2, **caractérisé en ce que** les (méth)acrylates d'alkyles comprennent de 25 à 35% en poids de méthacrylate de cétyle, de 55 à 65% en poids d'acrylate de stéaryle et de 5 à 15% en poids d'acrylate d'eicosyle.

4. Procédé selon l'une quelconque des revendications 1 à 3, **caractérisé en ce que** ladite composition est appliquée autour des yeux.

5. Procédé selon l'une quelconque des revendications 1 à 5, **caractérisé en ce que** ladite composition renferme de 0,1 à 10% en poids de tétrapolymère, par rapport au poids total de la composition.

6. Procédé selon la revendication 5, **caractérisé en ce que** la composition renferme de 0,2 à 5% en poids de tétrapolymère, par rapport au poids total de la composition.

7. Procédé selon l'une quelconque des revendications 1 à 6, **caractérisé en ce que** la composition renferme moins de 1% en poids de tensioactif, par rapport au poids total de la composition.

8. Procédé selon la revendication 7, **caractérisé en ce que** la composition renferme moins de 0,5% de tensioactif.

9. Procédé selon la revendication 7 ou 8, **caractérisé en ce que** la composition renferme en outre au moins un épaississant choisi parmi : les homo- et copolymères d'acide acrylique et les homo- et copolymères d'acrylamide et/ou d'acide 2-acrylamido-2-méthylpropane sulfonique.

10. Procédé selon l'une quelconque des revendications 1 à 9, **caractérisé en ce que** la composition renferme en outre au moins un composé choisi parmi : les agents desquamants ; les agents hydratants ; les agents dépigmentants ou propigmentants ; les agents anti-glycation ; les inhibiteurs de NO-synthase ; les agents stimulant la synthèse de macromolécules dermiques ou épidermiques et/ou empêchant leur dégradation ; les agents stimulant la prolifération des fibroblastes et/ou des kératinocytes ou stimulant la différenciation des kératinocytes ; les agents dermo-décontractant et/ou myorelaxants ; les autres agents tenseurs ; les agents anti-pollution et/ou anti-radicalaires ; les agents agissant sur la microcirculation ; les agents agissant sur le métabolisme énergétique des cellules ; et leurs mélanges.

11. Procédé selon la revendication 10, **caractérisé en ce que** ledit composé est choisi parmi : le rétinol et ses dérivés tels que le palmitate de rétinyle ; l'acide ascorbique et ses dérivés tels que l'ascorbyl phosphate de magnésium et le glucoside d'ascorbyle ; le tocophérol et ses dérivés tels que l'acétate de tocophéryle ; l'acide nicotinique et ses précurseurs tels que la nicotinamide ; l'ubiquinone ; le glutathion et ses précurseurs tels que l'acide L-2-oxothiazolidine-4-carboxylique ; les extraits de plantes et notamment les protéines végétales et leurs hydrolysats, ainsi que les phytohormones ; les extraits marins tels que les extraits d'algues ; les extraits bactériens ; les sapogénines telles que la diosgénine et les extraits de Wild Yam en contenant ; les céramides ; les α-hydroxyacides ; les β-hydroxyacides, tels que l'acide salicylique et l'acide n-octanoyl-5-salicylique ; le resvératrol ; les oligopeptides et pseudodipeptides et leurs dérivés acylés ; les sels de manganèse et de magnésium , en particulier les gluconates ; et leurs mélanges.

12. Utilisation cosmétique d'un tétrapolymère d'acide méthacrylique, de méthacrylate de méthyle, d'acrylate de butyle et de méthacrylate d'alkyles en C₁₆-C₂₀ en tant qu'agent tenseur de la peau humaine.

13. Utilisation selon la revendication 12, **caractérisée en ce** le tétrapolymère est destiné à lisser les rides et/ou estomper les signes cutanés de fatigue.
